# EUROPEAN PATENT SPECIFICATION

(11) **EP 3 833 781 B1**
(45) Date of publication and mention of the grant of the patent: **28.09.2022**
(21) Application number: 19769900.2
(22) Date of filing: 09.08.2019
(51) Int. Cl.: C12Q 1/6834

(54) **METHOD FOR DETECTING AND/OR QUANTIFYING A TARGET NUCLEOTIDE SEQUENCE**
VERFAHREN ZUM NACHWEISEN UND/ODER QUANTIFIZIEREN EINER ZIELNUKLEOTIDSEQUENZ
PROCÉDÉ DE DÉTECTION ET/OU DE QUANTIFICATION D'UNE SÉQUENCE NUCLÉOTIDIQUE CIBLE

(30) Priority: 09.08.2018 IT 201800008017
(43) Date of publication of application: 16.06.2021
(73) Proprietor: BIORIDIS S.R.L., 40124 Bologna (IT)
(72) Inventor: TORTORI, Andrea, 40124 Bologna (IT); ROSSI, Nicolò, 40124 Bologna (IT)
(74) Representative: Bosia, Alessandra
(86) International application number: PCT/IB2019/056785
(87) International publication number: WO 2020/031142

(56) References cited:
- WO-A1-2014/028793
- US-A1- 2016 046 988
- HIDEYUKI ARATA ET AL: "Rapid microRNA detection using power-free microfluidic chip: coaxial stacking effect enhances the sandwich hybridization", THE ANALYST, vol. 137, no. 14, 1 January 2012 (2012-01-01), page 3234, XP055571818, UK ISSN: 0003-2654, DOI: 10.1039/c2an16154k

### SEQUENCE LISTING

<110> Bioridis Srl
<120> METHOD AND KIT FOR DETECTING AND/OR QUANTIFYING A TARGET NUCLEOTIDE
   SEQUENCE
<130> 428-18
<160> **14**
<170> BiSSAP 1.3.6
<210> 1
   <211> 22
   <212> RNA
   <213> Artificial Sequence
<220>
   <223> RNA mir 146a fullmatch
<400> 1
   ugagaacuga auuccauggg uu 22
<210> 2
   <211> 22
   <212> RNA
   <213> Artificial Sequence
<220>
   <223> mir 146b mismatch
<400> 2
   ugagaacuga auuccauagg cu 22
<210> 3
   <211> 22
   <212> RNA
   <213> Artificial Sequence
<220>
   <223> 1 mismatch
<400> 3
   ugacaacuga auuccauggg uu 22
<210> 4
   <211> 22
   <212> RNA
   <213> Artificial Sequence
<220>
   <223> 4 mismatch
<400> 4
   ugagaacuga auuucacuug uu 22
<210> 5
   <211> 22
   <212> DNA
   <213> Artificial Sequence
<220>
   <223> DNA fullmatch
<400> 5
   tgagaactga attccatggg tt 22
<210> 6
   <211> 10
   <212> DNA
   <213> Artificial Sequence
<220>
   <223> PNA 1A
<220>
   <221> misc_feature
   <222> 1
   <223> /standard_name="N term acetilato"
<220>
   <221> misc_structure
   <222> 1..10
   <223> /standard_name="PNA"
<220>
   <221> misc_feature
   <222> 10
   <223> /standard_name="AEEA-K-CONH2"
<400> 6
   tcagttctca 10
<210> 7
   <211> 12
   <212> DNA
   <213> Artificial Sequence
<220>
   <223> PNA 1B
<220>
   <221> misc_feature
   <222> 1
   <223> /standard_name="N term acetilato"
<220>
   <221> misc_feature
   <222> 1..12
   <223> /standard_name="PNA"
<220>
   <221> misc_feature
   <222> 12
   <223> /standard_name="AEEA-K-CONH2"
<400> 7
   attcagttct ca 12
<210> 8
   <211> 16
   <212> DNA
   <213> Artificial Sequence
<220>
   <223> PNA 1C
<220>
   <221> misc_feature
   <222> 1
   <223> /standard_name="N term acetilato"
<220>
   <221> misc_feature
   <222> 1..16
   <223> /standard_name="PNA"
<220>
   <221> misc_feature
   <222> 16
   <223> /standard_name="AEEA-AEEA-K-CONH2"
<400> 8
   tggaattcag ttctca 16
<210> 9
   <211> 12
   <212> DNA
   <213> Artificial Sequence
<220>
   <223> PNA 1D
<220>
   <221> misc_feature
   <222> 1
   <223> /standard_name="NH2-AEEA-AEEA-AEEA"
<220>
   <221> misc_feature
   <222> 1..12
   <223> /standard_name="PNA"
<220>
   <221> misc_feature
   <222> 12
   <223> /standard_name="G-CONH2"
<400> 9
   actcttgact ta 12
<210> 10
   <211> 16
   <212> DNA
   <213> Artificial Sequence
<220>
   <223> PNA 2A
<220>
   <221> misc_feature
   <222> 1
   <223> /standard_name="N term acetilato - 5(K(Biot)-AEEA)"
<220>
   <221> misc_feature
   <222> 1..16
   <223> /standard_name="PNA"
<220>
   <221> misc_feature
   <222> 16
   <223> /standard_name="K-CONH2"
<400> 10
   aacccatgga attcag 16
<210> 11
   <211> 16
   <212> DNA
   <213> Artificial Sequence
<220>
   <223> PNA 2B
<220>
   <221> misc_feature
   <222> 1
   <223> /standard_name="N term acetilato - 5(K(Biot)-AEEA)"
<220>
   <221> misc_feature
   <222> 1..16
   <223> /standard_name="PNA"
<220>
   <221> misc_feature
   <222> 16
   <223> /standard_name="K-CONH2"
<400> 11
   catggaattc agttct 16
<210> 12
   <211> 12
   <212> DNA
   <213> Artificial Sequence
<220>
   <223> PNA 2C
<220>
   <221> misc_feature
   <222> 1
   <223> /standard_name="N term acetilato - 5(K(Biot)-AEEA)"
<220>
   <221> misc_feature
   <222> 1..12
   <223> /standard_name="PNA"
<220>
   <221> misc_feature
   <222> 12
   <223> /standard_name="K-CONH2"
<400> 12
   aacccatgga at 12
<210> 13
   <211> 16
   <212> DNA
   <213> Artificial Sequence
<220>
   <223> PNA 2D
<220>
   <221> misc_feature
   <222> 1
   <223> /standard_name="N term acetilato - 5(K(Biot)-AEEA"
<220>
   <221> misc_feature
   <222> 1..16
   <223> /standard_name="PNA"
<220>
   <221> misc_feature
   <222> 16
   <223> /standard_name="G-CONH2"
<400> 13
   ttttaaccca tggaat 16
<210> **14**
   <211> 12
   <212> DNA
   <213> Artificial Sequence
<220>
   <223> PNA 2E
<220>
   <221> misc_feature
   <222> 1
   <223> /standard_name="N term acetilato - 5(K(Biot)-AEEA)"
<220>
   <221> misc_feature
   <222> 1..12
   <223> /standard_name="DNA"
<220>
   <221> misc_feature
   <222> 12
   <223> /standard_name="KKK-CONH2"
<400> **14**
   aacccatgga at 12

## Description

### Cross-reference to related applications

This patent application claims priority from Italian patent application no. 102018000008017 filed on August 9, 2018, the entire disclosure of which is incorporated herein by reference.

### Technical field

The present invention relates to a method for detecting and/or quantifying a target nucleotide sequence in a sample based on a system with two nucleic acid probes.

### State of the art

The analysis of nucleic acids in different biological samples (biopsies, serum, plasma, cell cultures) is fundamental for the study and early diagnosis of many diseases (e.g. Alzheimer's, cancer, osteoarthritis, infectious diseases) in addition to having an increasing number of applications in the veterinary, agricultural and food sectors.

The nucleic acids of interest for analysis are genomic DNA, messenger RNA (mRNA), ribosomal RNA (rRNA), transport RNA (tRNA), heterogeneous nuclear RNA (hnRNA, pre-mRNA) and small sequences of RNA (small RNA). The small RNA are split into: microRNA (miRNA), small nuclear RNA (snRNA), short interfering RNA (siRNA), piwi-interacting RNA (piRNA).

The microRNA are the most interesting nucleic acids to analyse for the study and early diagnosis of many diseases. The microRNA are short sequences of RNA (ribonucleic acid) with 18-24 nucleotides and regulate the gene expression at transcriptional and post-transcriptional level, binding the target mRNA by inhibiting it. Very many miRNA in different species (in particular in mammals) have been described and often the difference in sequence (therefore effect) is due to the variation in an individual nucleotide. Their numerousness and their similarity require the development of highly specific methods able to recognize differences at the level of the individual nucleotide. MicroRNAs have the potential to be the next generation of biomarkers for the early diagnosis of numerous diseases. In fact, today many new biomarkers discovered are microRNAs, which are involved in the regulation of numerous biological systems and diseases, including various types of cancer, neurodegenerative and musculoskeletal diseases (e.g. osteoarthritis, Alzheimer's).

The main techniques used so far for the analysis of nucleic acids are PCR (polymerase chain reaction) and direct sequencing. These techniques require a long time to obtain results, highly qualified personnel to perform them and are very costly. Another limit of these techniques is their easy alteration by components present in the biological samples, for example Ca+, protease, nuclease and/or by the quality and type of buffers used to collect and extract the sample (for example concentration of EDTA, SDS). For this reason, another limit of these techniques is the preparation, extraction and purification of the sample prior to the actual analysis which tends to prolong times and increase costs. In particular, for microRNAs, the techniques used today (PCR and sequencing) and the products on sale are not satisfactory due to technologies that are complex, not easy to reproduce and are not specific enough (not optimized for short sequences), costly, require a long time and highly qualified personnel and are incompatible with the instruments and diagnostic-clinical workflow.

A method that avoids the use of PCR has been described in EP2639312. This method entails the use of oligonucleotides with DNA and antibodies able to recognise the DNA/RNA hybrids, but it still does not allow results to be obtained in a sufficiently simple, reproducible and sensitive manner.

Hideyuki A. et al., (2012), The Analyst, Vol. 137, No.14, p. 3234 discloses a method of detecting miRNAs comprising a sandwich hybridisation with a capture and a labeled fluorescent probe, wherein the two probes are perfectly adjacent so as to provide a coaxial stacking effect. This method displays a limited sensitivity.

An object of the present invention is therefore to provide a method for detecting and/or quantifying a target nucleotide sequence in a sample, which solves the above-mentioned problems in a simple and efficient manner. In particular the method must display high sensitivity, specificity, affinity, stability, ease of standardization, low cost and rapidity of execution.

This object is obtained by means of the present invention since it is relative to a method as defined in claim 1.

### Definitions

Unless defined otherwise, all the technical and scientific terms used here have the same meaning as the one commonly understood by a person normally skilled in the art to which this invention relates. Although in practice or in the evaluation of the present invention many materials and methods can be used similar or equivalent to those described herein, the preferred materials and methods are described below. Unless stated otherwise, the techniques described herein for use with the invention are standard methods well known to persons normally skilled in the art.

### Brief description of the Figures

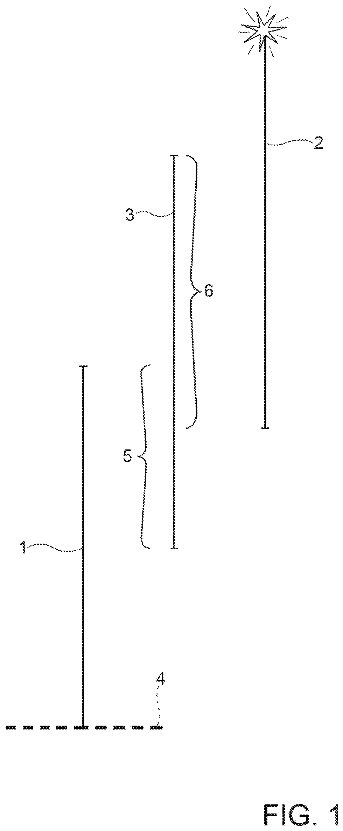

Figure 1 shows a schematic view of the elements used in the method according to the invention.

### Detailed disclosure of the invention

With reference to Figure 1, the method for detecting and/or quantifying a target nucleotide sequence 3 in a sample comprises the following steps.

In a first step the single strand target nucleotide sequence 3 is provided.

Prior amplification of the target nucleotide sequence 3 is not necessary, but it must be provided in the form of a single strand.

The target nucleotide sequence can be genomic DNA, messenger RNA (mRNA), ribosomal RNA (rRNA), transport RNA (tRNA), heterogeneous nuclear RNA (hnRNA, pre-mRNA) and small sequences of RNA (small RNA): microRNA (miRNA), small nuclear RNA (snRNA), short interfering RNA (siRNA), piwi-interacting RNA (piRNA).

Preferably, the target nucleotide sequence 3 is a microRNA (miRNA) .

Typically, the target nucleotide sequence 3 has a length from 10 to 50 nucleotides, preferably 18 to 30 nucleotides.

The sample that comprises the target nucleotide sequence 3 can consist of biological fluids including blood, serum, plasma, synovial liquid, saliva as is or diluted in buffer, and cell cultures, biopsies, tissues with a single homogenization and lysis phase without the need for extraction/purification and amplification phases. Preferably the sample is a living cell.

In a second step, the single strand target nucleotide sequence 3 is hybridized with a first probe 1 of single strand nucleic acid and with a second probe 2 of single strand nucleic acid. The first probe 1 is immobilized at one end, to a solid support 4 and comprises, at the opposite end, a sequence complementary to a first portion 5 of target nucleotide sequence 3.

The solid support 4 is selected from the group consisting of cellulose, nitrocellulose, acetate cellulose, crosslinked dextran, agarose, polystyrene, polypropylene, nylon, ceramic, glass, metal such as gold and silver, magnetite and combinations of the same. The solid support 4 can furthermore consist of cells, liposomes and vesicles of phospholipids. Preferably, the solid support 4 consists of microplates with 48, 96, 384 wells, magnetic beads, gold or silver beads, microparticles, strips, chromatography sheets, tubes, sticks, filter materials.

The probe 1 can be conjugated with molecules adapted to solid phase conjugation, for example: lysine, arginine, thiols, cysteine, glutathione.

The second probe 2 is marked at one end and comprises, at the opposite end, a sequence complementary to a second portion 6 of target nucleotide sequence 3.

The second probe 2 can be conjugated with one or more molecules, for example: glycine, lysine, arginine.

Preferably, the second probe 2 is marked by means of biotin, fluorescent molecules (for example cyanine, alexafluor), fluorescein.

Advantageously, the first portion 5 and the second portion 6 of target nucleotide sequence have 2 to 10 nucleotides in common.

The result of the second step is the formation of a first probe-target nucleotide sequence-second probe complex.

Preferably, the first probe 1 and the second probe 2 consist of a nucleic acid selected from the group consisting of PNA (peptide nucleic acid), LNA (locked nucleic acid), morpholino, orn-PNA (PNA with ornithine backbone), aep-PNA (aminoethylprolyl-PNA), aepone-PNA (aminoethyl pyrrolidone-PNA), GNA (glycol nucleic acid), HNA (hexitol nucleic acid), TNA (threose nucleic acid), ENA (2'-O,4'-C-ethylene-bridged nucleic acid), ANA (arabino nucleic acid), F-ANA (2'-F-arabino nucleic acid). Even more preferably, the first probe 1 and the second probe 2 consist of PNA (peptide nucleic acid), even more preferably PNA with a modified backbone structure wherein the alpha carbon has the side chain of the arginine or of the lysine as a substituent.

Using probes 1, 2 consisting of PNA allows the stability, specificity and sensitivity of the method to be increased. Similar characteristics can be obtained also with the use of other artificial oligonucleotides such as, for example, LNA and morpholino.

The non-charged nature of the PNA backbone is an important characteristic which means that the affinity bond between two strands of PNA and DNA or RNA is much stronger than the one between two stands of DNA or RNA, due to the absence of electrostatic repulsion. The greater stability of the double strands containing PNA translates into a higher melting temperature. PNA complexes with DNA or RNA are more sensitive to the presence of a single mismatch than the complexes formed of two strands of DNA or RNA. The PNA have a high biological stability; in fact, they are resistant to the attack of nucleases and proteases, both in human serum and in cells or in cell extracts, consequently extending their life time both in vitro and in vivo. The resistance to enzymatic degradation is accompanied by a high chemical stability. Due to these characteristics of the PNA and the possibility of operating at physiological pH (pH 7.2) as shown in example 3, the method can be used in living cells.

The method can also be used at saline concentrations between 0 mM and 1000 mM.

Preferably, the first probe 1 and/or the second probe 2 consist of a number of nucleotides from 10 to 18.

Preferably, when the method is used for detecting and/or quantifying miRNA, the probe 2 can comprise a region of 2-6 nucleotides additional to the N-terminal region to increase specificity only to the mature miRNA excluding the pre-miRNA, as in the SEQ ID NO: 13 (PNA 2d) where 4 Timine (T) are added to the terminal N region (see examples).

In a third step, the first probe-target nucleotide sequence-second probe complex is detected.

Preferably, the complex is detected by means of a system selected from the group consisting of streptavidin/antibody conjugated with: peroxidase enzyme (single HRP, HRP20, HRP40, HRP80), PE (phycoerythrin), a fluorescent molecule (for example cyanine, alexafluor, fluorescein).

A kit for use in the method according to the present invention comprises:
- a first probe 1 as described above;
- a second probe 2 as described above;
- optionally at least one buffer solution selected from the group consisting of SSC5x, SSC05x, SSC5x Tween 0.05/0.01%, SSC 0.5x Tween 0.05/0.01%, SSC2,5x SDS 1/0.5%, SSC2,5x SDS 0.1/0.05% BSA 5/10%, SSC5x SDS 0.1/0.05% BSA 5/10%, PBS, PBS Tween 0.05/0.1%, phosphate buffer between pH 5.8 and pH 7.2, phosphate buffer between pH 5.8 and pH 7.2. SDS 0.1/0.05%.

### Examples

The target nucleotide sequences 3 and the probes 1 and 2 used in the examples are described below.

Target nucleotide sequences. DNA and RNA from 22 single-strand bases, synthesis products purchased from Sigma-Aldrich. (The mismatch points are underlined)

| SEQ.ID.NO. | NAME | DESCRIPTION | Sequence 5'-3' |
|---|---|---|---|
| 1 | NA 1 | RNA mir 146a fullmatch | UGAGAACUGAAUUCCAUGGGUU |
| 2 | NA 2 | mir 146b mismatch | UGAGAACUGAAUUCC**A**UAGG**C**U |
| 3 | NA 3 | 1 mismatch | UGACAACUGAAUUCCAUGGGUU |
| 4 | NA 4 | 4 mismatches | UGAGAACUGAAUUUCACUUGUU |
| 5 | NA 5 | DNA fullmatch | TGAGAACTGAATTCCATGGGTT |

Probes 1 and 2 with PNA, produced by Bioridis with automatic peptide synthesis.

| SEQ.ID.NO. | NAME | Probe type | Sequence N term - C term | bases |
|---|---|---|---|---|
| 6 | PNA 1A | Probe 1 | ac-TCAGTTCTCA-AEEA-K- CONH2 | 10 |
| 7 | PNA 1B | Probe 1 | ac-ATTCAGTTCTCA-AEEA- K-CONH2 | 12 |
| 8 | PNA 1C | Probe 1 | ac-TGGAATTCAGTTCTCA- AEEA-AEEA-K-CONH2 | 16 |
| 9 | PNA 1D | Probe 1 | NH2-AEEA-AEEA-AEEA- ACTCTTGACTTA-Gly- CONH2 | 12 |
| 10 | PNA 2A | Probe 2 | ac-5(K(Biot)-AEEA)- AACCCATGGAATTCAG-K- CONH2 | 16 |
| 11 | PNA 2B | Probe 2 | ac-5(K(Biot)-AEEA)- CATGGAATTCAGTTCT-K- CONH2 | 16 |
| 12 | PNA 2C | Probe 2 | ac-5(K(Biot)-AEEA)- AACCCATGGAAT-K-CONH2 | 12 |
| 13 | PNA 2D | Probe 2 | ac-5(K(Biot)-AEEA)- TTTTAACCCATGGAAT-G- CONH2 | 16 |
| 14 | PNA 2E | Probe 2 | ac-5(K(Biot)-AEEA)- AACCCATGGAAT-KKK- CONH2 | 12 |

Key: K: lysine; G: Glycine; AEEA: Aminoethylethanolamine; ac: acetylate; K Biot: lysine with biotin conjugated on the side chain.

Preparation of solid phases. Two types of solid phases were tested with different conjugation systems to evaluate the capability of the method on different solid supports: a) Nunc Immobilizer Amino (Thermo-Fischer), microplates with 96 wells with a surface with a stable electrophilic group to bind positively charged groups; b) Nunc Maxisorp (Thermo-Fischer) microplates with 96 wells with a hydrophobic/hydrophilic surface adapted to bind many types of different molecules, electrically charged or neutral. The probes 1 were conjugated with the solid phases following the supplier's protocol.

Buffer. SSC5x (750 mM NaCl, 75 mM sc pH 7.0); SSC5x T (750 mM NaCl, 75 mM sc pH 7.0, Tween 0.05); SSC5x S (750 mM NaCl, 75 mM sc EDTA 0.5mM; SDS 0.05%, pH 7.0), SSC2.5x S (375 mM NaCl, 37.5 mM sc EDTA 0.5mM; SDS 0.05% pH 7.0;); SSC0.5x (75 mM NaCl, 7,5 mM sc pH 7.0); SSC0.5x T(75 mM NaCl, 7.5 mM sc pH 7.0; Tween 0.05); PBS (8.1mM Na₂HPO₄; KH₂PO₄ 1.7mM; KCl 0.27mM; NaCl 137mM; pH 7.2); PBST (8.1mM Na₂HPO₄; KH₂PO₄ 1.7mM; KCl 0.27mM; NaCl 137mM; pH 7.2; Tween 0.05/0.1); Phosphate buffer, BF5.8 (100mM NaP; 100mM NaCl EDTA 0.5mM pH 5.8) BF5.8 S (100mM NaP; 100mM NaCl EDTA 0.5mM SDS 0.05% pH 5.8); Phosphate buffer, BF7.2 (100mM NaP; 100mM NaCl EDTA 0.5mM pH 7.2); Phosphate buffer, BF7.2 S (100mM NaP; 100mM NaCl EDTA 0.5mM SDS 0.05% pH 7.2)

Detection system.

The detection systems tested in the examples and used in the method were: chemiluminescent, colorimetric and fluorescent, used following the guidelines of the suppliers' protocols. The data were acquired with a BIOTEK Synergy HT following the guidelines for use and for setting. The measurements were expressed in RLU (relative luminescence units) for the chemiluminescent system (maximum value 4.5*10⁶ RLU) and colorimetric system (maximum value 4.500 RLU) and in RFU (relative fluorescence unit, maximum value 99998 RFU) for the fluorescence (the results of the latter experiment are not shown).

### Example 1

Method. RNA NA1 were diluted in SSC 0.5 T0.1% at the concentration of 150fmol/ml. 100pl of the solution were plated in the wells, previously prepared with the probes and left to hybridize for 3 h. The RNA was then removed and the wells were washed. 10nM were added in BF5.8 with SDS 0.1% of the oligonucleotides for over-night (o.n) hybridization. The oligonucleotides were then removed and the wells were washed. 100ul were added in PBST of the SA-HRP40 at the concentration of 0.1 pg/ml for 60 min. The SA-HRP40 was then removed and the wells were washed. 100µl of chemiluminescent substrate (Clarity, Biorad) was added. Acquisitions performed after 5 min.

### Results

| Oligo probe 1 | Oligo probe 2 | Superimpos ition nucleotides | Delta (signal minus noise) Fullmatch NA 1 |
|---|---|---|---|
| 1a | 2c | 0 | 125909 |
| 1b | 2c | 2 | 1813352 |
| 1b | 2a | 6 | 456017 |
| 1c | 2c | 6 | 378015 |
| 1c | 2a | 10 | 208159 |

The example shows the advantage of the hybridization between probes 1 and 2 with the target probe 3 according to the nucleotides involved in the superimposition region.

### Example 2 (salt concentration influence)

Method. RNA NA1 was diluted in two parts in SSC 0.5 (NaCl 75mM) T0.1% and in SSC 5x (NaCl 750nM) T0.1% at the concentration of 150fmol/ml. 100pl of the solution were plated per well, previously prepared with the probe 1b and left to hybridize for 3 h. The RNA was then removed and the wells were washed. 10nM were added in BF5.8 and with SDS 0.1% of the probe 2c for over-night (o.n) hybridization. The oligonucleotides were then removed and the wells were washed. 100pl were added in PBST of the SA-HRP40 at the concentration of 0.1 pg/ml for 60min. The SA-HRP40 was then removed and the wells were washed. 100µl of chemiluminescent substrate (Clarity, Biorad) were added. Acquisitions performed after 5 min.

| Buffer | Delta (signal minus noise) |
|---|---|
| SSC0.5 | 777.338 |
| SSC 5 | 1.509.413 |

The example shows the ability of the method to work in wide ionic strength ranges (NaCl concentration between 750mM and 75mM).

### Example 3 (stability as pH varies)

Method. RNA NA1 was diluted in two parts in SSC 5x at the concentration of 150fmol/ml. 100µl of the solution were plated per well, previously prepared with the probe 1b and left to hybridize for 3 h. The RNA was then removed and the wells were washed. 10nM were added in BF5.8 or BF7.2 and with SDS 0.05% of the probe 2c for over-night (o.n) hybridization. The oligonucleotides were then removed and the wells were washed. 100µl were added in PBST of the SA-HRP40 at the concentration of 0.1 pg/ml for 60min. The SA-HRP40 was then removed and the wells were washed. 100µl of chemiluminescent substrate (Clarity, Biorad) were added. Acquisitions performed after 5 min.

| Buffer | Delta (signal minus noise) |
|---|---|
| BF5.8 | 802.804 |
| BF7.2 | 933.631 |

The example shows the ability of the method to work in wide pH ranges, in particular at physiological and cellular pH (pH 7.2) .

### Example 4 (reduced hybridization times, no overnight)

Method. RNA NA1 was diluted in two parts in SSC 5x at the concentrations of 150fmol/ml, 15fmol/ml, 1.5fmol/ml. 100µl of the solution were plated per well, previously prepared with the probe 1b and left to hybridize for 90min. The RNA was then removed and the wells were washed. 10nM were added in BF7.2 with SDS 0.05% of the probe 2c for the hybridization 2h. The oligonucleotides were then removed and the wells were washed. 100pl were added in PBST of the SA-HRP40 at the concentration of 0.1 pg/ml for 60min. The SA-HRP40 was then removed and the wells were washed. 100µl of chemiluminescent substrate (Clarity, Biorad) were added. Acquisitions performed after 5 min.

| RNA concentration | Delta (signal minus noise) |
|---|---|
| 150fmol/ml | off scale |
| 15fmol/ml | 1.082.246 |
| 1.5fmol/ml | 66.998 |

The example shows the ability of the method to work rapidly (5h) without the need for overnight phases.

### Example 5 (effect of positive charges)

Method. RNA NA1 was diluted in two parts in SSC 5x at the concentration of 15fmol/ml. 100µl of the solution were plated per well, previously prepared with the probe 1b and left to hybridize for 90 min. The RNA was then removed and the wells were washed. 10nM were added in BF7.2 with SDS 0.05% of the probe 2c or 2d or 2e for the hybridization 2h. The oligonucleotides were then removed and the wells were washed. 100pl were added in PBST of the SA-HRP40 at the concentration of 0.1 pg/ml for 60 min. The SA-HRP40 was then removed and the wells were washed. 100µl of chemiluminescent substrate (Clarity, Biorad) were added. Acquisitions performed after 5 min.

| Oligo probe 1 | Oligo probe 2 | Charges | Delta (signal minus noise) |
|---|---|---|---|
| 1c | 2d | 0 | 753855 |
| 1c | 2c | 1 | 725035 |
| 1c | 2e | 3 | 841788 |

The example shows the effect of the positive charges (inserted with the lysine), added to the sequence, on the hybridization capacity.

### Example 6 (use without extraction/purification in serum and living cells)

Method. The serum and the extract were treated with a RNase inhibitor (Thermo) and diluted 1:1 with SSC5x SDS 0.1% EDTA 1mM. RNA NA1 was diluted in parts at the concentration of 15fmol/ml. 100µl of the solution were plated per well, previously prepared with the probe 1b and left to hybridize for 90 min. The RNA was then removed and the wells were washed. 10nM were added in BF7.2 with SDS 0.05% of the probe 2c for hybridization 2h. The oligonucleotides were then removed and the wells were washed. 100µl were added in PBST of the SA-HRP40 at the concentration of 0.1 pg/ml for 60 min. The SA-HRP40 was then removed and the wells were washed. 100ul of chemiluminescent substrate (Clarity, Biorad) were added. Acquisitions performed after 5 min.

| Matrix | Delta (signal minus noise) |
|---|---|
| Ssc5x | 2038134 |
| serum | 2085434 |
| cells | 1214368 |

The example shows the ability of the method to work directly on biological samples: biological liquids (e.g. serum) or living cells without the need for extraction and/or purification phases.

### Example 7 (single step)

Method. RNA NA1 was diluted in two parts in SSC 5x at the concentration of 15fmol/ml. For one series, 100ul of the solution were plated per well, previously prepared with the probe 1b and left to hybridize for 90 min. The RNA was then removed and the wells were washed. In one series 10nM were added in BF7.2 with SDS 0.05% of probe 2c for the hybridization and in the other, a solution with 15fmol/ml of RNA NA1 with 10nM of 2c in BF7.2 with SDS 0.05% and left to hybridize for 2h. The oligonucleotides were then removed and the wells were washed. 100µl were added in PBST of the SA-HRP40 at the concentration of 0.1 pg/ml for 60 min. The SA-HRP40 was then removed and the wells were washed. 100pl of chemiluminescent substrate (Clarity, Biorad) were added. Acquisitions performed after 5 min.

| No. of steps | Delta (signal minus noise) |
|---|---|
| 2 steps (5h) | 1090550 |
| 1 step (3h) | 1227210 |

The example shows the ability of the method to work rapidly (3h) and with a single hybridization step.

### Example 8 (specificity of the method)

Method. RNA NA1, NA2, NA3, NA4 were diluted in parts at the concentration of 150fmol/ml in SSC2.5x S, 20nM were added of the probe 2d. 100pl of the solution were plated per well, previously prepared with the probe 1d and left to hybridize for 2h. The oligonucleotides were then removed and the wells were washed. 100pl were added in PBST of the SA-HRP40 at the concentration of 0.1 pg/ml for 60 min. The SA-HRP40 was then removed and the wells were washed. 100pl of chemiluminescent substrate (Clarity, Biorad) were added. Acquisitions performed after 5 min.

| RNA | Delta (signal minus noise) |
|---|---|
| NA1 | 2070630 |
| NA2 | 0 |
| NA3 | 0 |
| NA4 | 0 |

The example shows how the method is highly specific also at the level of a single mismatch.

### Example 9 (RNA and DNA comparison)

Method. RNA NA1, DNA NA5 were diluted in two parts at the concentration of 150fmol/ml in SSC2.5x S, 20nM were added of the probe 2d. 100pl of the solution were plated per well, previously prepared with the probe 1b and left to hybridize for 4h. The oligonucleotides were then removed and the wells were washed. 100pl were added in PBST of the SA-HRP40 at the concentration of 0.1 µg/ml for 60 min. The SA-HRP40 was then removed and the wells were washed. 100µl of chemiluminescent substrate (Clarity, Biorad) were added. Acquisitions performed after 5 min.

| Nucleic acid | Delta (signal minus noise) |
|---|---|
| NA1 (RNA) | 2300299 |
| NA5 (DNA) | 904829 |

The method can also be used to detect the DNA. The lower affinity for the DNA is advantageous for use with the RNA since it ensures a lower risk of aspecifics derived from any DNA fragments.

### Example 10 (colorimetric detection)

Method. RNA NA1 and NA2 were diluted in two parts at the concentration of 150fmol/ml and 15fmol/ml (only NA1) in SSC2.5x S, 20nM were added of the probe 2d. 100µl of the solution were plated per well, previously prepared with the probe 1b and left to hybridize for 4h. The oligonucleotides were then removed and the wells were washed. 100pl were added in PBST of the SA-HRP40 at the concentration of 0.1 pg/ml for 60 min. The SA-HRP40 was then removed and the wells were washed. 100pl of Colorimetric substrate (TMB High sensitivity substrate, Biolegend) were added. The reaction was blocked after 10 min. with the addition of 100ul of stop solution (Biolegend). The plate was read at 450nm according to the protocol.

| | Delta (signal minus noise) |
|---|---|
| NA1 150fmol/ml | 3.846 |
| NA1 15fmol/ml | 0.600 |
| NA2 150fmol/ml | 0.000 |

The method can also be used with the colorimetric detection and on a solid support different from the one used in the previous experiments.

### Example 11 (sensitivity)

Method. RNA NA1 was diluted in two parts at the concentrations of 150fmol/ml, 15fmol/ml 1.5fmol/ml, 150amol/ml, 15amol/ml, 1,5amol/ml, in SSC2.5x S, 10nM were added of the probe 2d. For each concentration, 100ul of the solution were plated per well, previously prepared with the probe 1b and left to hybridize for 2h. The oligonucleotides were then removed and the wells were washed. 10µl were added in PBST of the SA-HRP40 at the concentration of 0.1 pg/ml for 60 min. The SA-HRP40 was then removed and the wells were washed. 100µl of chemiluminescent substrate (Clarity, Biorad) were added. Acquisitions performed after 5 min.

The limit of detection (LOD) calculated as the noise +(3* standard deviation) is lower than the concentration of 1.5 amol/ml (last point of the example).

From the results shown in the examples, it is evident that the method according to the invention has high sensitivity, specificity, affinity, ease and rapidity of execution.

In particular, using partial superimpositions of three oligonucleotides (the two probes and the target nucleic acid) provides advantages that had previously never been obtained. In fact, the triplex complex has a greater stability than the duplex complex.

Lastly, it is clear that modifications and variations can be made to the method described and illustrated without departing from the protective scope of the attached claims.

## Claims

1. A method for detecting and/or quantifying a target nucleotide sequence (3) in a sample comprising the steps of:
- providing the target nucleotide sequence (3) as a single strand;
- hybridizing the single strand target nucleotide sequence (3) with a first probe (1) of single strand nucleic acid and with a second probe (2) of single strand nucleic acid,
the first probe (1) being immobilised at one end to a solid support (4) and comprising, at the opposite end, a sequence complementary to a first portion (5) of the target nucleotide sequence (3), the second probe (2) being marked at one end and comprising, at the opposite end, a sequence complementary to a second portion (6) of target nucleotide sequence (3), wherein the first portion (5) and the second portion (6) of target nucleotide sequence have 2 to 10 nucleotides in common,
so as to form a first probe-target nucleotide sequence- second probe complex;
- detecting the first probe-target nucleotide sequence- second probe complex.

2. The method according to claim 1, wherein the first probe (1) and the second probe (2) are formed by a nucleic acid selected from the group consisting of PNA (peptide nucleic acid), LNA (locked nucleic acid), morpholino, orn-PNA (PNA with an ornithine backbone), aep-PNA (aminoethylprolyl-PNA), aepone-PNA (aminoethyl pyrrolidone -PNA), GNA (glycolic nucleic acid), HNA (hexitol nucleic acid), TNA (threose nucleic acid), ENA (2'-O,4'-C-ethylene-bridged nucleic acid), ANA (arabino nucleic acid), F-ANA (2'-F-arabino nucleic acid).

3. The method according to claim 2, wherein the first probe (1) and the second probe (2) are formed by PNA (peptide nucleic acid).

4. The method according to any of the preceding claims, wherein the target nucleotide sequence (3) is a microRNA (miRNA).

5. The method according to any of the preceding claims, wherein the first probe (1) and/or the second probe (2) are formed by a number of nucleotides from 10 to 18.

6. The method according to any of the preceding claims, wherein the sample is a living cell.

7. The method according to any of the preceding claims, wherein the second probe (2) is marked by means of biotin, fluorescent molecules or fluorescein.

## Patentansprüche

1. Verfahren zum Nachweisen und/oder Quantifizieren einer Zielnucleotidsequenz (3) in einer Probe, umfassend die folgenden Schritte:
- Bereitstellen der Zielnucleotidsequenz (3) als ein Einzelstrang;
- Hybridisieren der Einzelstrang-Zielnucleotidsequenz (3) mit einer ersten Sonde (1) aus einer einzelsträngigen Nucleinsäure und mit einer zweiten Sonde (2) aus einer einzelsträngigen Nucleinsäure,
wobei die erste Sonde (1) an einem Ende an einem festen Träger (4) immobilisiert ist und am gegenüberliegenden Ende eine Sequenz umfasst, die komplementär zu einem ersten Bereich (5) der Zielnucleotidsequenz (3) ist, wobei die zweite Sonde (2) an einem Ende markiert ist und am gegenüberliegenden Ende eine Sequenz umfasst, die komplementär zu einem zweiten Bereich (6) der Zielnucleotidsequenz (3) ist, wobei der erste Bereich (5) und der zweite Bereich (6) der Zielnucleotidsequenz 2 bis 10 Nucleotide gemeinsam haben, so dass ein erste Sonde-Zielnucleotidsequenz-zweite Sonde-Komplex gebildet wird;
- Nachweisen des erste Sonde-Zielnucleotidsequenz-zweite Sonde-Komplexes.

2. Verfahren nach Anspruch 1, wobei die erste Sonde (1) und die zweite Sonde (2) durch eine Nucleinsäure gebildet sind, die aus der Gruppe ausgewählt ist, die besteht aus PNA (Peptid-Nucleinsäure), LNA (verbrücke Nucleinsäure), Morpholino, orn-PNA (PNA mit einem Ornithingerüst), aep-PNA (Aminoethylprolyl-PNA), aepone-PNA (Aminoethylpyrrolidon-PNA), GNA (Glykol-Nucleinsäure), HNA (Hexitol-Nucleinsäure), TNA (Threose-Nucleinsäure), ENA (2'-O,4'-C-Ethylen-verbrückte Nucleinsäure), ANA (Arabino-Nucleinsäure) und F-ANA (2'-F-Arabino-Nucleinsäure).

3. Verfahren nach Anspruch 2, wobei die erste Sonde (1) und die zweite Sonde (2) durch PNA (Peptid-Nucleinsäure) gebildet sind.

4. Verfahren nach einem der vorstehenden Ansprüche, wobei es sich bei der Zielnucleotidsequenz (3) um eine microRNA (miRNA) handelt.

5. Verfahren nach einem der vorstehenden Ansprüche, wobei die erste Sonde (1) und/oder die zweite Sonde (2) durch eine Anzahl von Nucleotiden von 10 bis 18 gebildet sind.

6. Verfahren nach einem der vorstehenden Ansprüche, wobei es sich bei der Probe um eine lebende Zelle handelt.

7. Verfahren nach einem der vorstehenden Ansprüche, wobei die zweite Sonde (2) mit Biotin, fluoreszierenden Molekülen oder Fluorescein markiert ist.

## Revendications

1. Méthode de détection et/ou de quantification d'une séquence nucléotidique cible (3) dans un échantillon comprenant les étapes suivantes :
- la fourniture de la séquence nucléotidique cible (3) sous forme de monobrin ;
- l'hybridation de la séquence nucléotidique cible (3) monobrin avec une première sonde (1) d'acide nucléique monobrin et avec une seconde sonde (2) d'acide nucléique monobrin,
la première sonde (1) étant immobilisée à une extrémité sur un support solide (4) et comprenant, à l'extrémité opposée, une séquence complémentaire à une première partie (5) de la séquence nucléotidique cible (3), la seconde sonde (2) étant marquée à une extrémité et comprenant, à l'extrémité opposée, une séquence complémentaire à une seconde partie (6) de la séquence nucléotidique cible (3), dans laquelle la première partie (5) et la seconde partie (6) de la séquence nucléotidique cible possèdent 2 à 10 nucléotides en commun,
de manière à former un complexe première sonde-séquence nucléotidique cible-seconde sonde ;
- la détection du complexe première sonde-séquence nucléotidique cible-seconde sonde.

2. Méthode selon la revendication 1, dans laquelle la première sonde (1) et la seconde sonde (2) sont formées par un acide nucléique sélectionné dans le groupe consistant en un APN (acide peptidonucléique), un ANB (acide nucléique bloqué), un morpholino, un orn-APN (APN avec un squelette d'ornithine), un aep-APN (aminoéthylprolyl-APN), un aepone-APN (aminoéthyl pyrrolidone-APN), un ANG (acide nucléique glycolique), un ANH (acide nucléique hexitol), un ANT (acide nucléique thréose), un ANE (acide nucléique à pont 2'-O, 4'-C-éthylène), un AAN (acide arabinonucléique), un F-AAN (acide 2'-F-arabinonucléique).

3. Méthode selon la revendication 2, dans laquelle la première sonde (1) et la seconde sonde (2) sont formées par un APN (acide peptidonucléique).

4. Méthode selon l'une quelconque des revendications précédentes, dans laquelle la séquence nucléotidique cible (3) est un microARN (miARN).

5. Méthode selon l'une quelconque des revendications précédentes, dans laquelle la première sonde (1) et/ou la seconde sonde (2) sont formées d'un nombre de nucléotides allant de 10 à 18.

6. Méthode selon l'une quelconque des revendications précédentes, dans laquelle l'échantillon est une cellule vivante.

7. Méthode selon l'une quelconque des revendications précédentes, dans laquelle la seconde sonde (2) est marquée au moyen d'une biotine, de molécules fluorescentes ou d'une fluorescéine.
